# EUROPEAN PATENT APPLICATION

(11) **EP 2 921 182 A1**
(43) Date of publication of application: **23.09.2015**
(21) Application number: 15159160.9
(22) Date of filing: 16.03.2015
(51) Int. Cl.: A61L 2/22, A47L 23/02, A47B 61/04

(54) **Sanitarizing device for item of clothing for sale and/or for rent**

(30) Priority: 19.03.2014 IT MI20140458
(71) Applicant: Allegrini S.p.A., 24050 Grassobbio (BG) (IT)
(72) Inventor: De Biasi, Giuseppe, 24050 Grassobbio (IT)
(74) Representative: Sgobba, Marco

(57) **Abstract**

A device (1) for disinfecting items of clothing comprises a first area (2) and a housing space (5) for the item of clothing. The housing space (5) comprises a base surface (5a) for receiving the item of clothing and a top surface (5b) opposite the base surface at which at least one dispensing nozzle (4) is arranged, configured to dispense a disinfecting aerosol inside the housing space (5). The device (1) also comprises a second area (3), distinct from the first (2), comprising a tank (10) for disinfecting liquid and an aerosol generating device (11) active on the tank (10), in fluid communication with the dispensing nozzle (4), to generate a disinfecting aerosol. The device (1) also comprises a command and control unit (15) configured to send at least activation and deactivation signals (SA, SD) to the aerosol generating device (11).

## Description

### TECHNICAL FIELD

The present invention refers to a device for disinfecting items of clothing.

The present invention is particularly useful for use in disinfecting items of clothing such as footwear, bowling shoes, climbing shoes, ski and snowboard boots, roller skates and ice skates and the like intended to be bought or rented by customers, although such uses only represent some examples of possible applications.

In the rest of the present description explicit reference will be made to ski boots as an example of an item of clothing for which the disinfecting device of the present invention is intended.

### BACKGROUND OF THE INVENTION

Sports enthusiasts in particular, and in any case any customer purchasing or renting items of clothing, pay particular attention to the condition and even more to the hygiene of the items that they wear before purchasing (to test their characteristics) or that they hire. A skier, for example, takes great care to see that his ski boots are properly cleaned and disinfected before use, both in terms of the shell and the outer ankle-piece (to highlight its decorative pattern) and in terms of the inner boot (for reasons of hygiene and comfort). In order to meet such requirements, on the market there are specific products for cleaning, or rather for disinfecting the inner part of the boot, in other words the boot part (i.e. the inner boot) that is in contact with the user's foot when the boot is worn. Usually, the operations of disinfecting the inside of the boot require a certain period in which it cannot be used, given for example the time needed to allow the inner boot of the boot to dry after it has been wetted or dampened, particularly in the freezing climate conditions of mountain environments. Therefore, it is not always possible to have a boot the inside of which has been perfectly disinfected, especially if the user forgets to take care of the cleaning operations as soon as he returns home, or in any case wherever he is staying, where the products for carrying out the disinfection are located.

Moreover, in the case of ski boots for rent in a shop, for example close to a ski resort, the inner part of the ski boot in direct contact with the user's foot becomes impregnated with sweat and remains as such until it is suitably disinfected by the renter. Therefore, it may be the case that many users wear the same boot before the renter disinfects it. Furthermore, in shops that sell ski boots, the latter are worn by the potential purchasers to test the fit and the functionality, as well as to see how it looks.

Therefore, it may be the case that a customer in the shop tries on a ski boot that has already been worn many times, with the consequent poor hygiene conditions.

Analogous examples can be quoted with reference to items of clothing such as footwear, bowling shoes, climbing shoes, ice skates and roller skates and the like. Indeed, such items of clothing are often for rent in dedicated sports centres, and, in any case, need to be worn in a shop in order for a potential customer to be able to appreciate and purchase them.

The Applicant has realized that it would be advantageous to be able to disinfect the items of clothing for sale or for hire whenever they are taken off, and in any case at times when the items of clothing are not being used.

The Applicant has also realized that it would be extremely advantageous to be able to disinfect items of clothing for sale or for rent whenever the user is forced to take a break, for example at meal time.

In this context, the technical task of the present invention is to propose a device for disinfecting items of clothing for sale or for rent capable of disinfecting them on demand, in other words upon specific request.

In particular, a purpose of the present invention is to provide a device for disinfecting items of clothing for sale or for rent that is capable of disinfecting them automatically and at any location.

Furthermore, a purpose of the present invention is to allow the customer using items of clothing for sale or for rent to immediately see that the item of clothing is perfectly disinfected.

### SUMMARY OF THE INVENTION

In accordance with the present invention, the technical task and the proposed purposes are achieved through a device for disinfecting items of clothing for sale or for rent according to the characteristics of one or more of the attached claims.

### BRIEF DESCRIPTION OF THE DRAWINGS

The characteristics and advantages of the present invention will become clearer from the following detailed description of an embodiment of a device for disinfecting items of clothing for sale or for rent in accordance with the present invention, given as a nonlimiting example with reference to the attached drawings, in which:
- figure 1 shows a schematic perspective view of a device for disinfecting items of clothing in accordance with the present invention,
- figure 2 shows a schematic front view of the device of figure 1,
- figure 3 represents a functional diagram of some parts of the distributor of figure 1.

### DETAILED DESCRIPTION OF THE INVENTION

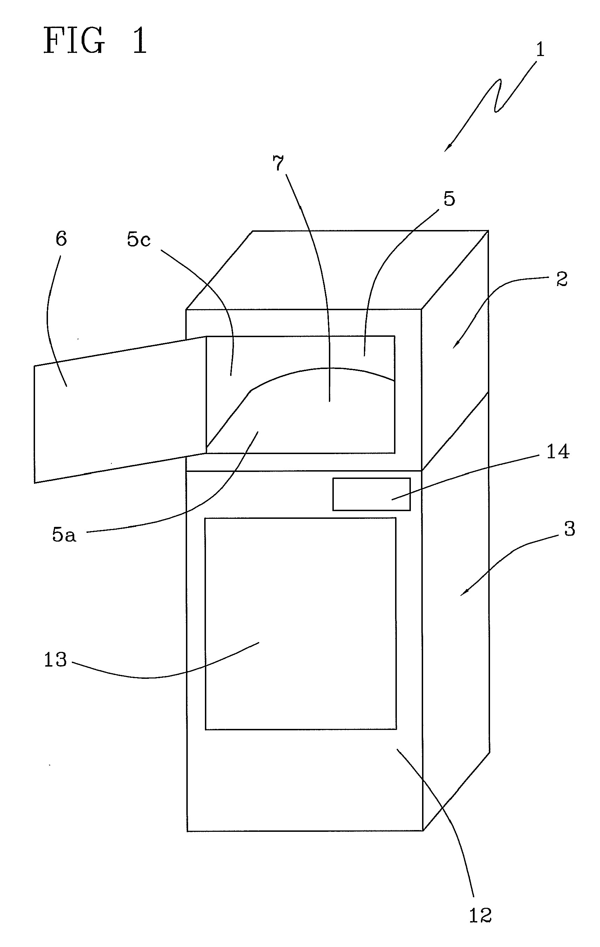

A device for disinfecting items of clothing for sale or for rent has been wholly indicated with reference numeral 1 in figure 1. In the rest of the present invention explicit reference will be made to ski boots as an example of an item of clothing for sale or for rent, without for this reason limiting the possible use of the device 1. The device 1 comprises two areas that are at least functionally separate from one another, and in particular a first area 2 and a second area 3. The first area 2 is configured to receive an item of clothing to be disinfected and comprises at least one dispensing nozzle 4 capable of dispensing a disinfecting aerosol. By disinfecting aerosol, in the context of the present invention, we mean the dispersion of a disinfecting liquid in a gas, for example air, in which the particles of disinfecting liquid have dimensions preferably comprised between 0.5 and 60 microns. The disinfecting liquid is a liquid capable of at least partially eliminating bacterial loads, algae, mould and bad odours. The disinfecting liquid can be of the type manufactured and used only with the authorization of the Ministry of Health (approved medical product) or of the type that is freely produced and sold.

The first area 2 comprises a housing space 5 for an item of clothing, for example a pair of ski boots, in which the item of clothing can be placed for the disinfecting operations. The housing space 5 comprises a base surface 5a configured to receive the item of clothing, a top surface 5b opposite the base surface 5a and preferably a side wall 5c that is substantially curved and has no sharp edges that extends between the base surface and the top surface. In an embodiment, the side wall 5c, in a plan view, comprises two substantially rectilinear segments, opposite one another and joined by an arc. In an embodiment, the housing space 5 is made from stainless steel in order to facilitate the cleaning and disinfecting operations thereof.

The housing space 5 is closed by a door 6 preferably arranged in the front area of the housing space 5.

The dispensing nozzle 4 is arranged inside the housing space 5, advantageously at the top surface thereof. In the preferred embodiment of the invention, the dispensing nozzle 4 is oriented in the housing space 5 so as to dispense the disinfecting aerosol downwards, in other words towards the base surface 5a of the housing space 5. In embodiments of the invention that are not illustrated, there can be more than one dispensing nozzle, each of which is arranged at the top surface 5b of the housing space 5. Preferably, the pressure with which the aerosol comes out from the dispensing nozzle 4 is slightly greater than atmospheric pressure, for example it is comprised, in absolute value, between 1.05 and 1.2 atm.

Inside the housing space 5, and in particular close to the base surface 5a, support members 7 for the item of clothing are also arranged, configured so as to stably hold the item of clothing during the activation of the dispensing nozzle 4. In particular, the support members 7 hold the items of clothing, like for example ski boots, so as to expose their inner part (in other words the part intended to come into contact with the user's foot) upwards. In this way, the inner part of the boot is directly exposed to the action of the dispensing nozzle 4. In other words, the dispensing nozzle 4 and the support members 7 cooperate to allow the disinfecting aerosol to reach and hit the inner part of the boot in a uniform manner. In an alternative embodiment not shown in the attached figures, the first area 2 comprises alternative and possibly removable support members configured to support an item of clothing (or the items of clothing) to be disinfected.

Preferably, the first area 2 comprises suction members 8 suitable for evacuating the disinfecting aerosol dispensed by the dispensing nozzle 4 from the housing space 5. Such suction members 8 can consist of a fan suitable for evacuating the aerosol from the housing space 5 to emit it into the external environment to the device 1 for disinfecting items of clothing.

Preferably, the first area 2 also comprises a filter (not illustrated) cooperating with the suction members 8 to filter the aerosol to be evacuated before it is freed into the external environment.

Preferably, the first area 2 comprises ventilation members 9 arranged inside the housing space 5 and suitable for promoting the uniform diffusion of the disinfecting aerosol dispensed by the dispensing nozzle 4.

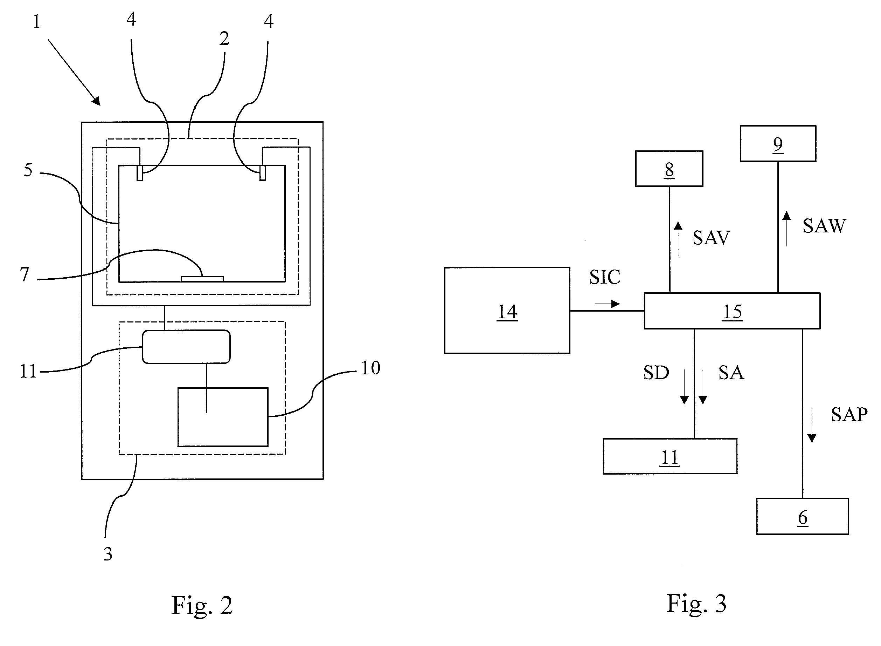

The second area 3 is configured to contain disinfecting liquid and to make disinfecting aerosol flow to the first area 2 and in particular to the dispensing nozzle 4. Preferably, the disinfecting liquid is diluted with water to 50% by volume. The second area 3 (schematised in figure 2) comprises a tank 10 for the disinfecting liquid and an aerosol generating device 11 active on the tank 10 to generate a disinfecting aerosol. The aerosol generating device 11 is in fluid communication with the dispensing nozzle 4, to dispense the disinfecting aerosol in the housing space 5. In the preferred embodiment of the invention, the aerosol generating device 11 is, preferably, an ultrasound nebulizer. The formation of the aerosol in the ultrasound nebulizer takes place by means of the contact of the disinfecting liquid with the surface of a piezoelectric crystal that vibrates at frequencies comprised between 20 kHz and 10 MHz, preferably 1.7 MHz. The ultrasound nebulizer is able to transform the disinfecting liquid into droplets with an average diameter of between 0.5 and 60 microns, preferably comprised between 1 and 10 microns, even more preferably comprised between 3 and 6 microns. The aerosol generating device 11 generates an aerosol that has a density Da that is greater than the density of air in the same pressure and temperature conditions, and in particular in ambient pressure and temperature conditions. Therefore, the aerosol dispensed by the dispensing nozzle 4 at the top surface 5b of the housing space 5, through the effect of gravity, tends to move downwards reaching the base surface 5a of the housing space. This allows an optimal diffusion and distribution of the disinfecting aerosol inside the housing space 5.

In an embodiment, the tank 10 for disinfecting liquid comprises a level sensor configured to signal when the minimum level of the disinfecting liquid has been reached, for example by switching on an LED and/or by emitting a sound and/or by signalling on a display. Preferably, such signalling systems are arranged in a position that is visible by a user so as to promote the timely refilling of the tank 10 for disinfecting liquid.

In the preferred embodiment of the invention, the second area 3 is a box-shaped body 12 defining an internal cavity containing the tank 10 for disinfecting liquid and the aerosol generating device 11. Such an internal cavity has an opening engaged by a door 13 suitable for allowing access to the cavity. In the embodiment shown in the attached figures, the first area 2 is arranged above the second area 3 so as to make a single containment structure. An embodiment that is not illustrated in the attached figures foresees that the first area 2 be laterally adjacent to the second area 3. In a further embodiment, the device 1 for disinfecting items of clothing comprises a plurality of first areas 2, where each first area 2 is configured to receive a respective item of clothing to be disinfected.

The aerosol generating device 11 is of the electric type. Concerning this, inside the second area 3 there are transformers able to be connected to the electrical energy distribution network that supply all of the electrical lines of the device 1 for disinfecting items of clothing, or it is possible to foresee electrical energy sources (like for example solar panels, accumulators, batteries and the like) directly housed inside the second area 3.

The device 1 for disinfecting items of clothing also comprises a payment interface 14 configured to activate a disinfecting cycle following a payment by a user. Such a payment interface can be of the type using tokens, prepaid cards, bank notes, credit card or a combination thereof. The payment interface 20 is configured to send a start of disinfecting cycle signal SIC to a command and control unit 15 following the payment by a user. In an alternative embodiment, the payment interface 14 can be replaced or added to by a different actuation system, for example of the type using buttons or through a touchscreen display or other systems that allow a disinfecting cycle to be started and interrupted.

Preferably, the command and control unit 15, schematized in figure 3, is a PLC and is configured to manage disinfecting cycles. The command and control unit 15 is configured to send activation and deactivation signals SA, SD to the aerosol generating device 11, to allow the dispensing nozzle 4 to dispense and to prevent the dispensing of disinfecting aerosol.

The activation signal SA is generated by the command and control unit 15 when a signal representative of the start of cycle SIC is received. Such a signal SIC is generated by the payment interface 14 or by an activation device like for example a button.

The command and control unit 15 is also configured to generate and send to the door 6 an opening signal SAP so that the door can be opened and access can be made inside the housing space 5.

In use, a user inserts the item of clothing into the first area 2, and in particular into the housing space 5. The door 6 is then closed. In the preferred embodiment of the invention, the user must proceed to pay for the service through the payment interface 14. Once payment has been made, the payment interface 14 generates the start of cycle signal SIC. In alternative embodiments, the closing of the door 6 or a dedicated button generate the start of cycle signal SIC. Once the start of cycle signal SIC has been received, the activation signal SA generated and sent by the command and control unit 15 activates the dispensing of the nozzle 4 for a predetermined time comprised between 10 and 90 seconds, preferably about 60 seconds. In any case, the activation time of the aerosol generating device 11 is such as to allow complete saturation of the housing space 5 with disinfecting aerosol. It should also be noted that the disinfecting liquid, when dispensed in the form of aerosol, does not wet, at least macroscopically, the item of clothing, allowing immediate use thereof at the end of the disinfecting cycle. In other words, the exposure of the item of clothing to the disinfecting aerosol does not require that the item of clothing be subjected to drying operations since the disinfecting aerosol is not able (both due to the time in which it stays in the housing space, and the amount dispensed) to significantly wet the item of clothing. Once such a predetermined activation time has passed, the command and control unit 15 sends the deactivation signal SD which stops the dispensing of the disinfecting aerosol. The disinfecting aerosol takes care of totally, or at least partially, eliminating bacteria from the part of the item of clothing in contact with the user's skin. In an embodiment, the deactivation signal SD can be sent after the door 6 has been opened during the disinfecting cycle by means of a sensor present on the door 6 or by pressing a stop cycle button.

Preferably, the command and control unit 15 generates and sends to the ventilation members 9 an activation signal SAW. The ventilation members 9 remain active for the entire duration of the dispensing of disinfecting aerosol by the dispensing nozzle 4. Such ventilation members 9 take care of forcing the circulation of the disinfecting aerosol inside the housing space 5. This operation allows optimal diffusion of the disinfecting aerosol inside the housing space 5. Thereafter, once a time of between 30 and 90 second has passed since the emission of the deactivation signal SD of the aerosol generating device 11, the command and control unit 15 is configured to send an activation signal SAV to the suction members 8, to activate them for a time comprised between 5 and 30 seconds. At this point, the command and control unit 15 generates the opening signal SAP and sends it to the door 6, which opens and allows the perfectly disinfected item of clothing to be taken out. It should be noted that the opening signal SAP of the door 6 is sent by the command and control unit 15 after a period of at least 30 second since the activation of the suction members 8. In an embodiment, following the opening of the door 6 while a disinfecting cycle is carried out, the command and control unit 15 is configured to send the activation signal SAV to the suction members 8 to suck the disinfecting aerosol from the housing space 5. The door 6 is suitable for allowing a user to visually monitor the inside of the housing space 5. Concerning this, the first area 2 comprises a plurality of LEDs suitable for lighting up the inside of the housing space 5 with at least two different colours. The two colours comprise a first colour indicative of the activation of a disinfecting cycle and a second colour indicative of the end of the disinfecting cycle.

It should be noted that the amount of disinfecting liquid consumed by the aerosol generating device 5 during a disinfecting cycle is comprised between 10 and 40 millilitres.

The invention achieves the proposed purposes, since the disinfecting device makes it possible to disinfect items of clothing, like for example bowling shoes, climbing shoes, ski and snowboard boots, roller skates and ice skates, in an automatic manner, in short times periods, efficiently and without wetting or dampening the item of clothing. The disinfecting device can for example be installed at ski resorts, ice skating rinks, climbing training centres, bowling alleys, in shops, in meal areas and in any case any place where a user goes to make a purchase, to hire out or simply to take a break.

When the disinfecting device is placed in a shop, the device can be used to disinfect the items of clothing that are worn by potential customers, so that every item of clothing on sale is always in perfect hygiene conditions. Advantageously, the disinfecting cycle can be carried out in short time periods and in the presence of the customer who, by witnessing the disinfecting procedure, is able to immediately see that the item of clothing is perfectly disinfected.

Clearly, those skilled in the art can bring numerous modifications and variants to the configurations described above, in order to satisfy contingent and specific requirements. For example, the two areas 2, 3 can be physically separate from each other, the tank 10 of disinfecting liquid can be arranged in a remote position with respect to the device, or furthermore the number of dispensing nozzles 4 can be whatever. Such variants and modifications are moreover all covered by the scope of protection of the invention as defined by the following claims.

## Claims

1. Device (1) for disinfecting items of clothing for sale or for rent comprising a first area (2) configured to receive an item of clothing to be disinfected and comprising a housing space (5) for said item of clothing, said housing space (5) comprising a base surface (5a) configured to receive said item of clothing and a top surface (5b) opposite said base surface (5a), said housing space (5) also comprising a front opening engaged by a door (6), suitable for allowing the insertion and extraction of said item of clothing from said housing space (5), said first area (2) also comprising at least one dispensing nozzle (4) positioned at said top surface (5b), arranged to dispense a disinfecting aerosol inside said housing space (5), said device (1) for disinfecting items of clothing also comprising a second area (3), distinct from the first (2), comprising a tank (10) for disinfecting liquid and an aerosol generating device (11) active on said tank (10) for disinfecting liquid, in fluid communication with said at least one dispensing nozzle (4), to generate disinfecting aerosol, said device (1) for disinfecting items of clothing also comprising a command and control unit (15) configured to send at least activation and deactivation signals (SA, SD) to said aerosol generating device (11).

2. Device (1) according to claim 1, wherein said housing space (5) comprises a substantially curved side wall (5c) that extends between said base surface (5a) and said top surface (5b).

3. Device (1) according to claim 1 or 2, wherein said aerosol generating device (11) is an ultrasound nebulizer.

4. Device (1) according to any one of the previous claims, wherein said first area (2) and said second area (3) are side-by-side or juxtaposed and are formed in a single containment structure.

5. Device (1) according to any one of the previous claims, wherein said first area (2) comprises suction members (8) suitable for evacuating said disinfecting aerosol dispensed by said at least one dispensing nozzle (4) from said housing space (5).

6. Device (1) according to claim 5, wherein said first area (2) comprises a filter cooperating with said suction members (8) to filter said disinfecting aerosol to be evacuated.

7. Device (1) according to any one of the previous claims, wherein said first area (2) comprises ventilation members (9) arranged inside said housing space (5) and suitable for forcing the circulation of said disinfecting aerosol dispensed by said at least one dispensing nozzle (4).

8. Device (1) according to any one of the previous claims, comprising a payment interface (14) configured to be active on said command and control unit (15) following payment by a user.

9. Device (1) according to any one of the previous claims, wherein said first area (2) comprises a plurality of LED lights suitable for illuminating the inside of said housing space (5) with at least two different colours, wherein a first colour is indicative of the activation of a disinfecting cycle and a second colour is indicative of the end of said disinfecting cycle.

10. Device (1) for disinfecting items of clothing according to any one of the previous claims, wherein said command and control unit (15) is configured to receive a signal representative of the start of cycle (SIC) and generate said activation signal (SA) upon receiving the start of cycle signal (SIC); said activation signal (SA) activating said aerosol generating device (11) for a predetermined time preferably comprised between 10 and 90 seconds.
